# EUROPÄISCHE PATENTANMELDUNG

(11) **EP 1 772 124 A2**
(43) Veröffentlichungstag der Anmeldung: **11.04.2007**
(21) Anmeldenummer: 06019030.3
(22) Anmeldetag: 12.09.2006
(51) Int. Cl.: A61F 9/02, C03C 4/00, G02C 11/04, C09K 11/00

(54) **Brille, insbesondere Arbeitsschutzbrille**

(30) Priorität: 06.10.2005 DE 102005047880
(71) Anmelder: Uvex Arbeitsschutz GmbH, 90766 Fürth (DE)
(72) Erfinder: Hermann, Herbert, 86736 Auhausen (DE); Hirschmann, Peter, Dr., 90768 Fürth (DE)
(74) Vertreter: Schneck, Herbert

(57) **Zusammenfassung**

Bei einer Brille, insbesondere Arbeitsschutz- oder Sportbrille, umfassend wenigstens eine Scheibe und einen Rahmen, ist vorgesehen, dass wenigstens ein Teil der Brille aus einem mit lumineszierenden Pigmenten versehenen Kunststoff besteht.

## Beschreibung

Die Erfindung richtet sich auf eine Brille, insbesondere eine Arbeitsschutzbrille.

Derartige Arbeitsschutzbrillen dienen dazu, die Augen von Arbeitern gegen Staub, Partikel, Säuren oder Licht zu schützen, wobei einerseits eine optimale Schutzfunktion und andererseits ein möglichst ungehinderter Durchblick angestrebt werden.

Hiervon ausgehend liegt der Erfindung die Aufgabe zugrunde, eine derartige Brille so weiterzubilden, dass Schutzfunktionen über den unmittelbaren Augenschutz hinaus realisierbar sind.

Diese Aufgabe wird erfindungsgemäß dadurch gelöst, dass wenigstens ein Teil der Brille aus einem mit lumineszierenden Pigmenten versehenen Kunststoff besteht.

Eine solche Brille hat im Arbeitsschutzbereich den Vorteil, dass die jeweiligen Brillenteile nachleuchten, wenn der Träger der Brille von einem gut beleuchteten Bereich in einen schlecht oder nicht beleuchteten Bereich wechselt, sodass der Brillenträger gut erkennbar und er hierdurch geschützt ist.

Eine derartige, mit nachleuchtenden Elementen ausgestattete Brille besitzt dementsprechend ein zusätzliches präventives, passives Sicherheitsmerkmal aufgrund der Erkennbarkeit bei diffusen Lichtverhältnissen, insbesondere bei Tätigkeiten in Bereichen, bei denen bei Stromausfall Dunkelheit entsteht, wie z. B. im Bergwerk, Tunnel oder Schacht. Im Sportbereich kann eine solche Brille vorteilhaft beim nächtlichen Radfahren oder beim Begehen von Höhlen eingesetzt werden. Schließlich ist eine Verwendung auch noch im Consumerbereich, wie z. B. in Diskotheken oder bei Open-Air-Events denkbar.

Insbesondere kann vorteilhafterweise bei einer mit Bügeln versehenen Brille vorgesehen sein, dass die Bügel einen derartigen lumineszierenden Kunststoff umfassen, sodass der Träger der Brille im Dunkeln praktisch durch zwei seitliche leuchtende Streifen markiert ist. Alternativ können natürlich auch andere Rahmenteile, wie Kupplungsteile oder der Mittelrahmen, lumineszierend ausgerüstet werden.

Günstigerweise ist der Kunststoff mit den lumineszierenden Pigmenten auf einen schlagzähen Grundkörper aufgebracht. Die Einbringung von lumineszierenden Pigmenten in einen Kunststoff führt nämlich dazu, dass dessen mechanische Festigkeitseigenschaften beeinträchtigt werden. Durch die Kombination mit einem schlagzähen Kunststoff-Grundkörper erhält man gleichwohl ein stabiles Produkt, wobei die Weichheit des mit den lumineszierenden Pigmenten versehenen Kunststoffes für den Träger zugleich vorteilhafte, hautfreundliche Oberflächeneigenschaften schafft.

Die nachleuchtende Überzugs-Hülle kann dementsprechend vorzugsweise aus weichem, transparenten Kunststoff, wie TPU oder TPE bzw. Silikon, bestehen. Es sind andererseits aber auch harte Überzugs-Komponenten, wie PP, PE, PS, CP, CA, PA und PC möglich.

Der Kunststoff mit den lumineszierenden Pigmenten kann im Zweikomponenten-Spritzgießverfahren aufgebracht sein, wobei insbesondere ein Monosandwich- oder Gegenstromspritzverfahren in Betracht kommt. Die großen Vorteile dieser Verfahren für die Herstellung von Brillenbügeln aus unterschiedlichen Kunststoffen werden in der nicht vorveröffentlichten deutschen Patentanmeldung 10 2005 034 081 beschrieben.

Als lumineszierende Pigmente kommen insbesondere Aluminate in Betracht, mit welchen eine Nachleuchtdauer von bis zu acht Stunden erreicht werden kann.

Es können generell alle für Brillenfassungen geeigneten Kunststoffe mit lumineszierenden Pigmenten dotiert werden. Bevorzugt sind transparente Werkstoffe, besonders bevorzugt sind PA 12 transparent und PC. Als schlagzäher Grundkörper, der nicht notwendigerweise transparent sein muss, kommen PC, PC-Blends (wie PC/PBT, PC/ABS, PC/TBU), Polyamide sowie PA-Blends oder POM in Frage. Eine besonders geeignete Kombination besteht aus einem tragenden schlagzähen Grundkörper aus PC/PBT mit lumineszierendem PC-Überzug.

Nachfolgend wird die Erfindung anhand eines bevorzugten Ausführungsbeispiels näher erläutert:
- Es handelt sich um eine Arbeitsschutzbrille mit einer einzigen durchgehenden Scheibe, einem Rahmen und zwei an den Rahmen angelenkten Bügeln.
- Der schlagzähe Grundkörper besteht aus PC/PBT.
- Der Grundkörper ist im Zweikomponenten-Monosandwichverfahren gemäß DE 10 2005 034 081 mit einem Überzug versehen.
- Der Überzug ist bezogen auf den Tragezustand der Brille in horizontaler Richtung zwischen 0,8 und 1,2 mm und in vertikaler Richtung zwischen 2,0 und 3,0 mm dick.
- Der Überzug besteht aus PC.
- Der Überzug ist mit Aluminaten als lumineszierende Pigmente versehen.

## Patentansprüche

1. Brille, insbesondere Arbeitsschutz- oder Sportbrille, umfassend wenigstens eine Scheibe und einen Rahmen, **dadurch gekennzeichnet, dass** wenigstens ein Teil der Brille aus einem mit lumineszierenden Pigmenten versehenen Kunststoff besteht.

2. Brille nach Anspruch 1, **dadurch gekennzeichnet, dass** die Brille mit Bügeln versehen ist und die Bügel einen lumineszierenden Kunststoff umfassen.

3. Brille nach Anspruch 1 oder 2, **dadurch gekennzeichnet, dass** der Kunststoff mit den lumineszierenden Pigmenten auf einen schlagzähen Grundkörper aufgebracht ist.

4. Brille nach Anspruch 3, **dadurch gekennzeichnet, dass** der Kunststoff mit den lumineszierenden Pigmenten auf einen Kunststoff-Grundkörper im Zweikomponenten-Spritzgießverfahren aufgebracht ist.

5. Brille nach Anspruch 3, **dadurch gekennzeichnet, dass** der Bügel durch ein Monosandwich- oder Gegenstromspritzverfahren hergestellt ist.

6. Brille nach Anspruch 1, **dadurch gekennzeichnet, dass** die lumineszierenden Pigmente Aluminate sind.

7. Brille nach Anspruch 1, **dadurch gekennzeichnet, dass** der Kunststoff mit den lumineszierenden Pigmenten aus PA 12 transparent oder PC besteht.

8. Brille nach Anspruch 1, **dadurch gekennzeichnet, dass** der Kunststoff mit den lumineszierenden Pigmenten aus einer harten Komponente wie PP, PE, PS, CP, CA, PA und vorzugsweise PC besteht.

9. Brille nach Anspruch 3, **dadurch gekennzeichnet, dass** der tragende, schlagzähe Grundkörper aus PC/PC-Blends, wie PC/PBT, PC/ABS, PC/TPU, Polyamiden sowie PA-Blends oder POM besteht.
